# EUROPÄISCHE PATENTSCHRIFT

(11) **EP 1 339 672 B1**
(45) Veröffentlichungstag und Bekanntmachung des Hinweises auf die Patenterteilung: **06.10.2004**
(21) Anmeldenummer: 01984785.4
(22) Anmeldetag: 29.11.2001
(51) Int. Cl.: C07C 253/34, C07C 209/84

(54) **VERFAHREN ZUR REDUZIERUNG DES GEHALTS AN EINEM UNGESÄTTIGTEN AMIN IN EINER MISCHUNG ENTHALTEND EIN AMIN UND EIN NITRIL**
METHOD FOR REDUCING THE CONTENT OF AN UNSATURATED AMINE IN A MIXTURE CONTAINING AN AMINE AND A NITRILE
PROCEDE DE REDUCTION DE LA TENEUR EN UNE AMINE INSATUREE D'UN MELANGE CONTENANT UNE AMINE ET UN NITRILE

(30) Priorität: 30.11.2000 DE 10059716
(43) Veröffentlichungstag der Anmeldung: 03.09.2003
(73) Patentinhaber: BASF AKTIENGESELLSCHAFT, 67056 Ludwigshafen (DE)
(72) Erfinder: OHLBACH, Frank, 40219 Düsseldorf (DE); BENISCH, Christoph, 69214 Eppelheim (DE); LUYKEN, Hermann, 67069 Ludwigshafen (DE); ANSMANN, Andreas, 69168 Wiesloch (DE); FISCHER, Rolf-Hartmuth, 69121 Heidelberg (DE); MELDER, Johann-Peter, 67459 Böhl-Iggelheim (DE); BASSLER, Peter, 68519 Viernheim (DE); MAIXNER, Stefan, 68723 Schwetzingen (DE)
(86) Internationale Anmeldenummer: PCT/EP2001/013954
(87) Internationale Veröffentlichungsnummer: WO 2002/044135

(56) Entgegenhaltungen:
- EP-A- 0 497 333
- EP-A- 0 502 439
- WO-A-93/14064
- WO-A-93/16984
- WO-A-98/34900
- WO-A-98/34912
- J. KOSKIKALLIO : "Nucleophilic Reactivity" ACTA CHEMICA SCANDINAVICA, Bd. 23, Nr. 5, 1969, Seiten 1477-1489, XP001064740 in der Anmeldung erwähnt

## Beschreibung

Die vorliegende Erfindung betrifft ein Verfahren zur Reduzierung des Gehalts an einem aliphatischen, einfach ungesättigten Amin (III) in einer Mischung (IV) enthaltend ein Aminonitril (I) oder ein Diamin (II) oder deren Gemische und Amin (III), dadurch gekennzeichnet, daß man
a) Mischung (IV) mit einem anionischen Nukleophil (V),
   das ein nukleophiles Atom ausgewählt aus der Gruppe bestehend aus Sauerstoff, Stickstoff und Schwefel aufweist,
   das zur Aufnahme eines H⁺-Ions fähig ist unter Bildung einer Säure mit einem pKₐ-Wert im Bereich von 7 bis 11, gemessen in Wasser bei 25°C, und
   das eine relative Nukleophilie, gemessen in Methylperchlorat/Methanol bei 25°C,
   im Bereich von 3,4 bis 4,7 im Falle von Sauerstoff als nukleophilem Atom,
   im Bereich von 4,5 bis 5,8 im Falle von Stickstoff als nukleophilem Atom und
   im Bereich von 5,5 bis 6,8 im Falle von Schwefel als nukleophilem Atom
   aufweist,
   in einer Menge im Bereich von 0,01 bis 10 Mol pro Mol an Amin (III) in Mischung (IV) unter Erhalt einer Mischung (VI) umsetzt und
b) Aminonitril (I) oder Diamin (II) oder deren Gemische von Mischung (VI) bei einer Temperatur im Bereich von 50 bis 170°C und einem Druck im Bereich von 0,5 bis 100 kPa abdestilliert.

Mischungen enthaltend ein Aminonitril oder ein Diamin oder deren Gemische und ein ungesättigtes Amin, wobei im Sinne der vorliegenden Erfindung unter einem ungesättigten Amin eine zyklische oder lineare Verbindung enthaltend mindestens eine Kohlenstoff-Stickstoff-Doppelbindung oder eine Verbindung, die mindestens eine Kohlenstoff-Stickstoff-Doppelbindung, beispielsweise durch eine Eliminierungsreaktion, bilden kann, verstanden wird, fallen üblicherweise bei der partiellen Hydrierung von Dinitrilen zu Aminonitrilen oder einem Gemisch aus Aminonitrilen und Diaminen oder bei der vollständigen Hydrierung von Dinitrilen zu Diaminen an.

Die partielle Hydrierung von Adipodinitril (ADN) unter gleichzeitiger Herstellung von Hexamethylendiamin (HMD) und 6-Aminocapronitril (ACN), sowie die vollständige Hydrierung von ADN zu HMD, in Gegenwart eines Katalysators auf der Basis eines Metalls wie Nickel, Cobalt, Eisen, Rhodium oder Ruthenium ist allgemein bekannt, beispielsweiwe aus: K.Weissermel, H.-J.Arpe, Industrielle Organische Chemie, 3.Auflage, VCH Verlagsgesellschaft mbH, Weinheim, 1988, Seite 266, US-A 4 601 859, US-A 2 762 835, US-A 2 208 598, DE-A 848 654, DE-A 954 416, DE-A 42 35 466, US-A 3 696 153, DE-A 19500222, WO-A-92/21650 und DE-A-19548289.

Als Nebenprodukte entstehen unter anderem Azepin-Derivate wie N-(2-Azepano)-1,6-Diaminohexan und N-(2-Azepano)-6-Aminocapronitril, insbesondere 2-Aminoazepan und Tetrahydroazepin (THA).

Diese Azepin-Derivate, die wegen ihrer Farbgebung und Verschlechterung der Produkteigenschaften unerwünschte Verunreinigungen der üblicherweise für die Herstellung von Kunstfasern oder technischen Kunststoffen verwendeten Aminonitrile und Diamine darstellen, lassen sich nur mit erheblichem Aufwand von den Aminonitrilen, Diaminen oder deren Gemischen abtrennen.

Aus der WO-A-98/34900 und der WO-A-98/34912 ist ein Verfahren zur Reduzierung des Gehaltes an Tetrahydroazepin (THA) in einer Mischung enthaltend 6-Aminocapronitril bekannt.

EP-A-497333 beschreibt die Trennung von aliphatischen Aminonitrilen oder aliphatischen Diaminen aus Mischungen, die ein aliphatisches Aminonitril oder aliphatisches Diamin und ein zyklisches, aliphatisches, einfach ungesättigtes Amin enthalten, durch Zugabe von Basen, wobei die Base im stöchiometrischen Überschuß bezogen auf das zyklische, aliphatische, einfach ungesättigte Amin einzusetzen ist. Dabei werden als Basen Alkalihydroxide, Eralkalihydroxide, Tetraalkylammoniumhydroxid, Alkalialkoxide und Erdalkalialkoxide empfohlen.

Nachteilig bei diesem Verfahren ist eine gleichzeitig stattfindende Polymerisation von Wertprodukt, die zu einem erheblichen Verlust an Wertprodukt und zu unerwünschten Ablagerungen in den zu der Ausübung des Verfahrens eingesetzten Apparaturen und Maschinen führt.

Aufgabe der vorliegenden Erfindung war es daher, ein Verfahren bereitzustellen, das die Reduzierung des Gehalts an einem aliphatischen, einfach ungesättigten Amin in einer Mischung, enthaltend ein Aminonitril oder Diamin oder deren Gemische und ein aliphatisches, einfach ungesättigtes Amin, auf technisch einfache und wirtschaftliche Weise unter Vermeidung der genannten Nachteile ermöglicht.

Demgemäß wurde das eingangs definierte Verfahren gefunden.

Als Aminonitril (I) kommen Verbindungen mit einer oder mehreren, wie zwei, drei oder vier, vorzugsweise einer Nitrilgruppe in Betracht, insbesondere solche, die mindestens eine Nitrilgruppe aufweisen, die in Nachbarschaft zu einem aliphatischen Kohlenstoffatom steht, welches einen oder zwei, vorzugsweise zwei Wasserstoffatome trägt, oder Gemische solcher Aminonitrile.

Als Aminonitril (I) kommen Verbindungen mit einer oder mehreren, wie zwei, drei oder vier, vorzugsweise einer Aminogruppe in Betracht, insbesondere solche, die mindestens eine Aminogruppe aufweisen, die in Nachbarschaft zu einem aliphatischen Kohlenstoffatom steht, welches einen oder zwei, vorzugsweise zwei Wasserstoffatome trägt, oder Gemische solcher Aminonitrile. Besonders bevorzugt sind Aminonitrile mit endständiger, also am Ende einer Alkylkette befindlichen, Aminogruppe.

Aminonitril (I) basiert vorzugsweise auf einem Alkyl-Gerüst.

Aminonitril (I) weist in einer bevorzugten Ausführungsform 4 bis 12 Kohlenstoff-Atome auf.

Als Aminonitril (I) kommen vorzugsweise Aminonitrile ausgewählt aus der Gruppe bestehend aus 4-Aminobutyronitril, 5-Aminovaleronitril, 2-Methyl-5-aminovaleronitril, 6-Aminocapronitril, 12-Aminododecanitril, insbesondere 6-Aminocapronitril, in Betracht.

Die Herstellung solcher Aminonitrile kann in an sich bekannter Weise erfolgen.

6-Aminocapronitril kann durch partielle katalytische Hydrierung mit einem molekularen Wasserstoff enthaltenden Gas von ADN zu Mischungen, die HMD und ACN enthalten, erhalten werden.

Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannte Umsetzung sind beispielsweise in WO-A-96/20166, DE-A-19636768 und DE-A-19646436 beschrieben.

Als Diamin (II) kommen Verbindungen mit zwei oder mehr, wie zwei, drei oder vier, vorzugsweise zwei Aminogruppen in Betracht, insbesondere solche, die mindestens zwei Aminogruppen aufweisen, die in Nachbarschaft zu einem aliphatischen Kohlenstoffatom stehen, welches ein oder zwei, vorzugsweise zwei Wasserstoffatome trägt, besonders bevorzugt Diamine mit endständigen, also am Ende einer Alkylkette befindlichen Aminogruppen oder Gemische solcher Diamine.

Diamin (II) basiert vorzugsweise auf einem Alkyl-Gerüst.

Diamin (II) weist in einer bevorzugten Ausführungsform 4 bis 12 Kohlenstoff-Atome auf.

Als Diamin (II) kommen vorzugsweise Diamine ausgewählt aus der Gruppe bestehend aus 1,4-Diaminobutan, 1,5-Diaminopentan, 2-Methyl-1,5-diaminopentan, 1,6-Diaminohexan (HMD), 1,12-Diaminododecan in Betracht.

Die Herstellung solcher Diamine kann in an sich bekannter Weise erfolgen.

HMD kann man durch partielle katalytische Hydrierung mit einem molekularen Wasserstoff enthaltenden Gas von ADN zu Mischungen, die HMD und ACN enthalten, oder durch vollständige Hydrierung mit einem molekularen Wasserstoff enthaltenden Gas von ADN erhalten werden.

Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannten Umsetzungen können beispielsweise den bereits oben genannten Veröffentlichungen entnommen werden.

Als Amine (III) kommen zyklische oder lineare Verbindungen enthaltend mindestens eine Kohlenstoff-Stickstoff-Doppelbindung oder eine Verbindung, die mindestens eine Kohlenstoff-Stickstoff-Doppelbindung, beispielsweise durch eine Eliminierungsreaktion, bilden kann, oder Gemische solcher Verbindungen in Betracht.

Vorteilhaft kann man als Amin (III) eine Verbindung der Formel

R¹-(CH₂)ₙ-CH=N-(CH₂)ₘ-R²

wobei
- n, m: unabhängig voneinander 1, 2, 3, 4, 5, 6, 7, 8, 9, 10, 11 oder 12 vorzugsweise 4, 5 oder 6 und
- R¹,R²: unabhängig voneinander -CN oder -NH₂ darstellen
oder der Formel 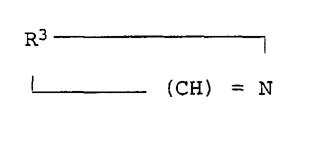 wobei
- R³: einen Alkenylrest mit 3, 4, 5, 6, 7, 8, 9, 10, 11 zu dem Ringsystem gehörigen Kohlenstoffatomen darstellt

In einer bevorzugten Ausführungsform setzt man ein Amin (III) ausgewählt aus der Gruppe bestehend aus, Dihydropyrrol, Tetrahydropyridin, 3-Methyl-Tetrahydropyridin, Tetrahydroazepin und Mono-ungesättigte Cyclododecylamine oder deren Gemische ein.

Diese Amine (III) können in der Mischung (IV) als einzelne Verbindungen oder als Addukte, beispielsweise an ein Nitril (I), insbesondere Aminonitril, vorliegen, wobei diese Addukte im Sinne der vorliegenden Erfindung ebenfalls als Amin (III) bezeichnet werden.

Solche Amine (III) sowie Verfahren zu deren Herstellung sind allgemein bekannt. So kann Tetrahydroazepin bei der partiellen katalytischen Hydrierung mit einem molekularen Wasserstoff enthaltenden Gas von ADN zu Mischungen, die HMD und ACN enthalten, in der Regel in Mengen von 1 bis 10000 ppm bezogen auf das Gemisch nach den für die Herstellung der ACN beschriebenen Verfahren in Mischungen (IV) erhalten werden.

Ebenso können die genannten Amine (III) durch Oxidation von Aminen, wie HMD, beispielsweise mit molekularen Sauerstoff enthaltenden Gasen, gebildet werden.

In einer bevorzugten Ausführungsform kann man als Mischung (IV) den aus der partiellen katalytischen Hydrierung, wie Gasphasenhydrierung oder Flüssigphasenhydrierung, mit einem molekularen Wasserstoff enthaltenden Gas von Dinitrilen, insbesondere ADN, in Gegenwart eines Katalysators, wie eines Suspensionskatalysators oder Festbettkatalysators, erhaltenen Reaktionsaustrag, der im Falle von ADN als Ausgangsverbindung ACN als Aminonitril (I), HMD als Diamin (II) und Tetrahydroazepin als Amin (III) enthält, einsetzen, wobei gewünschtenfalls zuvor bei der Hydrierung gegebenenfalls eingesetztes Lösungsmittel teilweise oder vollständig abgetrennt werden kann. Nach den bisherigen Beobachtungen kann es vorteilhaft sein, einen bei der Hydrierung eingesetzten Katalysator vor dem Einsatz von Mischung (IV) in dem erfindungsgemäßen Verfahren abzutrennen.

In einer bevorzugten Ausführungsform kann man als Mischung (IV) den aus der vollständigen katalytischen Hydrierung, wie Gasphasenhydrierung oder Flüssigphasenhydrierung, mit einem molekularen Wasserstoff enthaltenden Gas von Dinitrilen, insbesondere ADN, in Gegenwart eines Katalysators, wie eines Suspensionskatalysators oder Festbettkatalysators, erhaltenen Reaktionsaustrag, der im Falle von ADN als Ausgangsverbindung HMD als Diamin (II) und Tetrahydroazepin als Amin (III) enthält, einsetzen, wobei gewünschtenfalls zuvor bei der Hydrierung gegebenenfalls eingesetztes Lösungsmittel teilweise oder vollständig abgetrennt werden kann. Nach den bisherigen Beobachtungen kann es vorteilhaft sein, einen bei der Hydrierung eingesetzten Katalysator vor dem Einsatz von Mischung (IV) in dem erfindungsgemäßen Verfahren abzutrennen.

Erfindungsgemäß versetzt man Mischung (IV) mit einem anionischen Nukleophil (V).

Unter dem Begriff anionisch wird im Sinne der vorliegenden Erfindung verstanden, daß Nukleophil (V) in Summe einfach oder mehrfach, wie zwei oder dreifach, vorzugsweise einfach negativ geladen ist.

Unter dem Begriff nukleophil wird im Sinne der vorliegenden Erfindung die in Koskikallo, Acta Chem. Scand. 23 (1969) Seiten 1477-1489 beschriebene Fähigkeit einer Verbindung verstanden, in methanolischer Lösung aus Methylperchlorat bei 25°C die Perchlorat-Gruppe zu verdrängen, wobei die verbleibende Methylgruppe über ein nukleophiles Atom von Verbindung (V) an Verbindung (V) gebunden wird.

Als nukleophiles Atom von Verbindung (V) kommt ein Atom ausgewählt aus der Gruppe bestehend aus Stickstoff, Sauerstoff und Schwefel, vorzugsweise Stickstoff oder Sauerstoff in Betracht.

Erfindungsgemäß ist Verbindung (V) zur Aufnahme eines H⁺-Ions fähig unter Bildung einer Säure mit einem pKₐ-Wert im Bereich von 7 bis 11, vorzugsweise 8 bis 10,5, gemessen in Wasser bei 25°C.

Erfindungsgemäß weist Verbindung (V) eine relative Nukleophilie, gemessen in Methylperchlorat/Methanol bei 25°C gemäß Koskikallo, Acta Chem. Scand. 23 (1969) Seiten 1477-1489 und bestimmt gemäß Seite 1487-1488, im Bereich von 3,4 bis 4,7 vorzugsweise 3,6 bis 4,6, im Falle von Sauerstoff als nukleophilem Atom, im Bereich von 4,5 bis 5,8 vorzugsweise 4,8 bis 5,7 im Falle von Stickstoff als nukleophilem Atom und im Bereich von 5,5 bis 6,8 vorzugsweise 5,8 bis 6,7 im Falle von Schwefel als nukleophilem Atom auf.

Im Falle von Sauerstoff als nukleophilem Atom von (V) kommen vorteilhaft Phenolate in Betracht, wobei das aromatische Ringsystem des Phenolats einfach oder mehrfach, wie zwei- oder dreifach substituiert sein kann, beispielsweise durch eine C₁- bis C₄-Alkylgruppe, wie Methyl, Ethyl, n-Propyl, i-Propyl, n-Butyl, i-Butyl, s-Butyl, t-Butyl, durch Halogen, wie Fluor, Chlor, Brom, Jod, durch eine Nitrogruppe, durch eine Estergruppe, durch eine Carbonylgruppe oder durch eine Aminogruppe.

Im Falle von Stickstoff als nukleophilem Atom von (V) kommen vorteilhaft Verbindungen mit der strukturellen Einheit

(R⁴ R⁵ N) ⁻

mit
- R⁴:: Rest einer organischen, aliphatischen, arylaliphatischen oder arylischen Säure, vorzugsweise Carbonsäure- oder Sulfonsäuregruppe, wobei der Rest R⁴ substituiert sein kann wie bereits oben für Phenolat beschrieben,
- R⁵:: Rest einer organischen, aliphatischen, arylaliphatischen oder arylischenSäure, vorzugsweise Carbonsäure- oder Sulfonsäuregruppe, oder Wasserstoff oder eine C₁- bis C₄-Alkylgruppen, wie Methyl, Ethyl, n-Propyl, i-Propyl, n-Butyl, i-Butyl, s-Butyl, t-Butyl, wobei der Rest R⁵ substituiert sein kann wie bereits oben für Phenolat beschrieben
wobei R⁴ und R⁵ außerhalb des in der obigen Formel genannten Stickstoffs miteinander verknüpft sein können, beispielsweise über eine Alkylen-, Alkylarylen- oder eine Arylenbrücke, vorzugsweise über eine Arylenbrücke,
in Betracht.

In einer bevorzugten Ausführungsform kann als Nukleophil (V) ein Lactam-Anion eingesetzt werden der allgemeinen Formel 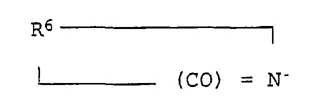 wobei
- R⁶: einen Alkylenrest mit 3, 4, 5, 6, 7, 8, 9, 10, 11 zu dem Ringsystem gehörigen Kohlenstoffatomen darstellt, wobei der Rest R⁶ substituiert sein kann wie bereits oben für Phenolat beschrieben.

In einer bevorzugten Ausführungsform kann als Nukleophil (V) Caprolactam-Anion eingesetzt werden.

In einer weiteren besonders bevorzugten Ausführungsform kann als Nukleophil (V) Benzolsulfonamid-Anion eingesetzt werden.

In einer weiteren besonders bevorzugten Ausführungsform kann als Nukleophil (V) Phthalimid-Anion eingesetzt werden.

In einer weiteren besonders bevorzugten Ausführungsform kann als Nukleophil (V) Phenolat eingesetzt werden.

Zum Ausgleich der negativen Ladung des anionischen Nukleophils (V) kann man dieses zusammen mit einem oder mehreren Kationen, vorzugsweise ausgewählt aus der Gruppe bestehend aus Lithium, Natrium, Kalium, Rubidium, Cäsium, Magnesium, Calcium, insbesondere bestehend aus Lithium, Natrium, Kalium, Magnesium, Calcium, besonders bevorzugt bestehend aus Natrium und Kalium einsetzen.

Erfindungsgemäß versetzt man Mischung (IV) mit Nukleophil (V) in einer Menge im Bereich von 0,01 bis 10 Mol pro Mol an Amin (III) in Mischung (IV).

Vorteilhaft kann die Menge an Nukleophil (V) mindestens 0,05, insbesondere 0,1 Mol pro Mol an Amin (III) in Mischung (IV) betragen.

Vorteilhaft kann die Menge an Nukleophil (V) höchstens 1, insbesondere höchstens 0,8, besonders bevorzugt höchstens 0,5 Mol pro Mol an Amin (III) in Mischung (IV) betragen.

Die Zugabe von Nukleophil (V) zu Mischung (IV) kann in an sich bekannter Weise, beispielsweise in üblichen Mischapparaturen, wie Behälter, Produktleitungen und Mischeinrichtungen, erfolgen unter Erhalt einer Mischung (VI).

Die Zugabe von Nukleophil (V) zu Mischung (IV) kann vor der Zuführung der Mischung (VI) in eine Destillationsvorrichtung zur Abtrennung von Nitril (I) aus der Mischung (VI) erfolgen. Dabei haben sich durchschnittliche mittlere Kontaktzeiten von Mischung (IV) und Nukleophil (V) vor der Zuführung in eine Destillationsvorrichtung von 5 bis 120 Minuten, insbesondere 10 bis 60 Minuten als vorteilhaft erwiesen. Dabei kommen vorteilhaft Temperaturen im Bereich vo 50 bis 170°C in Betracht.

Ebenso ist es möglich, Mischung (IV) und Nukleophil (V) einer solchen Vorrichtung getrennt zuzuführen und die Umsetzung von Mischung (IV) mit Nukleophil (V) und die Abtrennung von Nitril (I) aus der Mischung (VI) in einem Verfahrensschritt durchzuführen. Dabei kann Nukleophil (V) auf den Kopf, über die gesamte Höhe auf eine der Trennstufen oder in den Sumpf der Destillationsvorrichtung zugegeben werden.

Erfindungsgemäß destilliert man Nitril (I) von Mischung (VI) bei einer Temperatur im Bereich von 50 bis 170°C, vorzugsweise von 70 bis 150 und einem Druck im Bereich von 0,5 bis 100 kPa, vorzugsweise von 0,5 bis 10 kPa ab.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen, Füllkörperkolonnen oder Seitenabzugskolonnen oder verfahrenstechnische Varianten solcher Apparaturen.

Die Destillation kann man in mehreren, wie 2 oder 3 Kolonnen, vorteilhaft einer einzigen Kolonne durchführen.

Aminonitrile und Diamine stellen Vorstufen zur Herstellung von technisch wichtigen Polyamiden, wie Nylon 6 oder Nylon 6.6 dar.

### Beispiele

### Beispiel 1

500 ml 6-Aminocapronitril mit einem Tetrahydroazepin (THA) -Gehalt von 300 Gew.-ppm wurden mit 30 mol-%, bezogen auf THA, Kaliumphthalimid versetzt und über eine 1m lange Destillationskolonne mit V2A-Drahtnetzringen bei 96°C und 0,5 kPa (Sumpf temperatur 110-115 °C) destilliert. Nachdem 450 ml überdestilliert waren, wurde die Destillation abgebrochen. Im Destillat wurden 6 ppm THA gefunden.

### Beispiel 2

500 ml 6-Aminocapronitril mit einem THA-Gehalt von 1 Gew.-% wurden mit 50 mol-%, bezogen auf THA, Kaliumphthalimid versetzt und über eine 1m lange Destillationskolonne mit V2A-Drahtnetzringen bei 96°C und 0,5 kPa (Sumpftemperatur 110-115 °C) destilliert. Nachdem 450 ml überdestilliert waren, wurde die Destillation abgebrochen. Im Destillat wurden 0,09 Gew.-% THA gefunden.

### Beispiel 3

Zur kontinuierlichen Abtrennung von THA aus 6-Aminocapronitril wurde 6-Aminocapronitril mit einem THA-Gehalt von 300 Gew.-ppm in einen 750 ml-Behälter kontinuierlich gepumpt, in dem eine Suspension von Kaliumphthalimid in 6-Aminocapronitril mit einem THA-Gehalt von 300 Gew.-ppm bei 65°C gerührt wurde. Dabei stellte sich ein Kaliumphthalimid-Gehalt der Lösung von 160-170 Gew.-ppm ein.

Diese Lösung wurde kontinuierlich aus dem Behälter in einen 250 ml-Destillationskolben gepumpt, aus dem das ACN bei 10 mbar und 118 °C Sumpftemperatur über eine 30 cm lange Kolonne mit V2A-Drahtnetzringen abdestilliert wurde.

Bei einer Belastung von 210 ml/h, einem Abnahme-/Rücklaufverhältnis von 50:50 und einer Sumpfausschleusung von 10 ml/h wurden im Destillat 30 Gew.-ppm THA gefunden. Die Ausbeute an 6-Aminocapronitril betrug 95%.

## Patentansprüche

1. Verfahren zur Reduzierung des Gehalts an einem zyklischen oder linearen Amin (III), enthaltend mindestens eine Kohlenstoff-Stickstoff-Doppelbindung oder an einer Verbindung die mindestens eine Kohlenstoff-Stickstoff-Doppelbindung bilden kann, in einer Mischung (IV) enthaltend ein Aminonitril (I) oder ein Diamin (II) oder deren Gemische und Amin (III), **dadurch gekennzeichnet, daß** man
a) Mischung (IV) mit einem anionischen Nukleophil (V),
das ein nukleophiles Atom ausgewählt aus der Gruppe bestehend aus Sauerstoff, Stickstoff und Schwefel aufweist,
das zur Aufnahme eines H⁺-Ions fähig ist unter Bildung einer Säure mit einem pKₐ-Wert im Bereich von 7 bis 11, gemessen in Wasser bei 25°C, und
das eine relative Nukleophilie, gemessen in Methylperchlorat/Methanol bei 25°C,
im Bereich von 3,4 bis 4,7 im Falle von Sauerstoff als nukleophilem Atom,
im Bereich von 4,5 bis 5,8 im Falle von Stickstoff als nukleophilem Atom und
im Bereich von 5,5 bis 6,8 im Falle von Schwefel als nukleophilem Atom
aufweist,
in einer Menge im Bereich von 0,01 bis 10 Mol pro Mol an Amin (III) in Mischung (IV) unter Erhalt einer Mischung (VI) umsetzt und
b) Aminonitril (I) oder Diamin (II) oder deren Gemische von Mischung (VI) bei
einer Temperatur im Bereich von 50 bis 170°C und einem Druck im Bereich von 0,5 bis 100 kPa abdestilliert.

2. Verfahren nach Anspruch 1, wobei man als Aminonitril (I) ein aliphatisches Aminonitril mit 4 bis 12 C-Atomen einsetzt.

3. Verfahren nach Anspruch 1, wobei man als Aminonitril (I) ein aliphatisches Aminonitril mit 4 bis 12 C-Atomen ausgewählt aus der Gruppe bestehend aus 4-Aminobutyronitril, 5-Aminovaleronitril, 2-Methyl-5-aminovaleronitril, 6-Aminocapronitril, 12-Aminododecanitril einsetzt.

4. Verfahren nach Anspruch 1, wobei man als Diamin (II) ein aliphatisches Diamin mit 4 bis 12 C-Atomen einsetzt.

5. Verfahren nach Anspruch 1, wobei man als Diamin (II) ein aliphatisches Diamin mit 4 bis 12 C-Atomen ausgewählt aus der Gruppe bestehend aus 1,4-Diaminobutan, 1,5-Diaminopentan, 2-Methyl-1,5-diaminopentan, 1,6-Diaminohexan, 1,12-Diaminododecan einsetzt.

6. Verfahren nach den Ansprüchen 1 bis 5, wobei man als Amin (III) eine Verbindung ausgewählt aus der Gruppe bestehend aus, Dihydropyrrol, Tetrahydropyridin, 3-Methyl-Tetrahydropyridin, Tetrahydroazepin, 2-Aminoazepan, N-(2-Azepano)-1,6-Diaminohexan, N-(2-Azepano)-6-Aminocapronitril und Mono-ungesättigte Cyclododecylamine einsetzt.

7. Verfahren nach den Ansprüchen 1 bis 6, wobei man als Nukleophil (V) Benzolsulfonamid-Anion einsetzt.

8. Verfahren nach den Ansprüchen 1 bis 7, wobei man als Nukleophil (V) Phthalimid-Anion einsetzt.

9. Verfahren nach den Ansprüchen 1 bis 8, wobei man als Nukleophil (V) Phenolat einsetzt.

10. Verfahren nach den Ansprüchen 1 bis 9, wobei man als Nukleophil (V) ein Lactam-Anion einsetzt der allgemeinen Formel 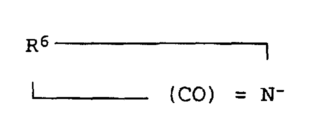 wobei R⁶ einen Alkylenrest mit 3, 4, 5, 6, 7, 8, 9, 10, 11 zu dem Ringsystem gehörenden Kohlenstoffatomen darstellt.

11. Verfahren nach den Ansprüchen 1 bis 10, wobei man als Nukleophil (V) Caprolactam-Anion einsetzt.

12. Verfahren nach den Ansprüchen 1 bis 11, wobei man anionisches Nukleophil (V) zusammen mit einem Kation ausgewählt aus der Gruppe bestehend aus Lithium, Natrium, Kalium, Magnesium und Calcium einsetzt.

## Claims

1. A process for reducing the content of a cyclic or linear amine (III) containing at least one carbon-nitrogen double bond, or of a compound capable of forming at least one carbon-nitrogen double bond, in a mixture (IV) containing an aminonitrile (I) or a diamine (II), or mixtures thereof, and the amine (III), wherein
a) the mixture (IV) is reacted with an anionic nucleophile (V),
which contains a nucleophilic atom selected from the group comprising oxygen, nitrogen and sulfur,
which is capable of taking up an H⁺ ion to form an acid with a pKₐ ranging from 7 to 11, measured in water at 25°C, and
which has a relative nucleophilicity, measured in methyl perchlorate/methanol at 25°C,
ranging from 3.4 to 4.7 when oxygen is the nucleophilic atom,
ranging from 4.5 to 5.8 when nitrogen is the nucleophilic atom, and
ranging from 5.5 to 6.8 when sulfur is the nucleophilic atom,
in an amount ranging from 0.01 to 10 mol per mole of amine (III) in the mixture (IV), to give a mixture (VI), and
b) the aminonitrile (I) or the diamine (II), or mixtures thereof, are distilled from the mixture (VI) at a temperature ranging from 50 to 170°C and a pressure ranging from 0.5 to 100 kPa.

2. A process as claimed in claim 1 wherein the aminonitrile (I) used is an aliphatic aminonitrile having from 4 to 12 C atoms.

3. A process as claimed in claim 1 wherein the aminonitrile (I) used is an aliphatic aminonitrile having from 4 to 12 C atoms selected from the group comprising 4-aminobutyronitrile, 5-aminovaleronitrile, 2-methyl-5-aminovaleronitrile, 6-aminocapronitrile and 12-aminododecanenitrile.

4. A process as claimed in claim 1 wherein the diamine (II) used is an aliphatic diamine having from 4 to 12 C atoms.

5. A process as claimed in claim 1 wherein the diamine (II) used is an aliphatic diamine having from 4 to 12 C atoms selected from the group comprising 1,4-diaminobutane, 1,5-diaminopentane, 2-methyl-1,5-diaminopentane, 1,6-diaminohexane and 1,12-diaminododecane.

6. A process as claimed in any of claims 1 to 5 wherein the amine (III) used is a compound selected from the group comprising dihydropyrrole, tetrahydropyridine, 3-methyltetrahydropyridine, tetrahydroazepine, 2-aminoazepan, N-(2-azepano)-1,6-diaminohexane, N-(2-azepano)-6-aminocapronitrile and monounsaturated cyclododecylamines.

7. A process as claimed in any of claims 1 to 6 wherein the nucleophile (V) used is a benzenesulfonamide anion.

8. A process as claimed in any of claims 1 to 7 wherein the nucleophile (V) used is a phthalimide anion.

9. A process as claimed in any of claims 1 to 8 wherein the nucleophile (V) used is phenate.

10. A process as claimed in any of claims 1 to 9 wherein the nucleophile (V) used is a lactam anion of the general formula 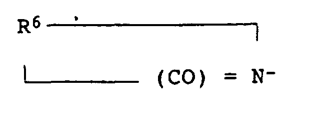 in which R⁶ is an alkylene radical having 3, 4, 5, 6, 7, 8, 9, 10 or 11 carbon atoms belonging to the ring system.

11. A process as claimed in any of claims 1 to 10 wherein the nucleophile (V) used is a caprolactam anion.

12. A process as claimed in any of claims 1 to 11 wherein the anionic nucleophile (V) is used together with a cation selected from the group comprising lithium, sodium, potassium, magnesium and calcium.

## Revendications

1. Procédé de réduction de la teneur en une amine cyclique ou linéaire (III), contenant au moins une double liaison carbone - azote, ou en un composé pouvant former au moins une double liaison carbone - azote, dans un mélange (IV) contenant un aminonitrile (I) ou une diamine (II) ou leurs mélanges, et une amine (III), **caractérisé en ce que**
a) on fait réagir avec un mélange (IV) un nucléophile anionique (V),
qui présente un atome nucléophile choisi dans le groupe formé par l'oxygène, l'azote et le soufre,
qui est capable de capter un ion H⁺ avec formation d'un acide d'un pKₐ de l'ordre de 7 à 11, mesuré dans l'eau à 25°C, et
qui présente une nucléophilie relative, mesurée dans du perchlorate de méthyle / méthanol à 25°C,
de l'ordre de 3,4 à 4,7 dans le cas d'hydrogène en tant qu'atome nucléophile,
de l'ordre de 4,5 à 5,8 dans le cas d'azote en tant qu'atome nucléophile,
de l'ordre de 5,5 à 6,8 dans le cas de soufre en tant qu'atome nucléophile,
en une quantité de l'ordre de 0,01 à 10 moles par mole d'amine (III) dans le mélange (IV), avec obtention d'un mélange (VI), et
b) on sépare d'aminonitrile (I) ou la diamine (II) par distillation, à une température de l'ordre de 50 à 170°C et une pression de l'ordre de 0,5 à 100 kPa.

2. Procédé selon la revendication 1, dans lequel on met en oeuvre en tant qu'aminonitrile (I) un aminonitrile aliphatique comportant de 4 à 12 atomes de C.

3. Procédé selon la revendication 1, dans lequel on met en oeuvre un aminonitrile aliphatique (I) comportant de 4 à 12 atomes de C choisi dans le groupe formé par le 4-aminobutyronitrile, le 5-aminovaléronitrile, le 2-méthyl-5-aminovaléronitrile, le 6-aminocapronitrile, le 12-aminododécanitrile.

4. Procédé selon la revendication 1, dans lequel on met en oeuvre en tant que diamine (II) une diamine aliphatique comportant de 4 à 12 atomes de C.

5. Procédé selon la revendication 1, dans lequel on met en oeuvre en tant que diamine (II) une diamine aliphatique comportant de 4 à 12 atomes de C choisie dans le groupe formé par le 1,4-diaminobutane, le 1,5-diaminopentane, le 2-méthyl-1,5-diaminopentane, le 1,6-diaminohexane, le 1,12-diaminododécane.

6. Procédé selon l'une quelconque des revendications 1 à 5, dans lequel on met en oeuvre en tant qu'amine (III) un composé choisi dans le groupe formé par le dihydropyrrole, la tétrahydropyridine, la 3-méthyl-tétrahydropyridine, la tétrahydroazépine, le 2-aminoazépane, le N-(2-azépano-1,6-diaminohexane, le N-(2-azépano)-6-aminocapronitrile et des cyclododécylamines monoinsaturées.

7. Procédé selon l'une quelconque des revendications 1 à 6, dans lequel on met en oeuvre en tant que nucléophile (V) un anion benzène-sulfonamide.

8. Procédé selon l'une quelconque des revendications 1 à 7, dans lequel on met en oeuvre en tant que nucléophile (V) un anion phtalimide.

9. Procédé selon l'une quelconque des revendications 1 à 8, dans lequel on met en oeuvre en tant que nucléophile (V) un phénolate.

10. Procédé selon l'une quelconque des revendications 1 à 9, dans lequel on met en oeuvre en tant que nucléophile (V) un anion lactame de la formule générale 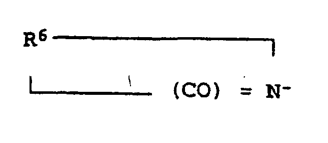 dans laquelle R⁶ représente un radical alkylène comportant 3, 4, 5, 6, 7, 8, 9, 10, 11 atomes de carbone appartenant au système cyclique.

11. Procédé selon l'une quelconque des revendications 1 à 10, dans lequel on met en oeuvre en tant que nucléophile (V) un anion caprolactame.

12. Procédé selon l'une quelconque des revendications 1 à 11, dans lequel on met en oeuvre le nucléophile anionique (V) ensemble avec un cation choisi dan le groupe formé par le lithium, le sodium, le potassium, le magnésium et le calcium.
